# EUROPEAN PATENT SPECIFICATION

(11) **EP 2 663 655 B1**
(45) Date of publication and mention of the grant of the patent: **02.09.2015**
(21) Application number: 12702895.9
(22) Date of filing: 13.01.2012
(51) Int. Cl.: C12Q 1/68

(54) **PAIRED END RANDOM SEQUENCE BASED GENOTYPING**
AUF ZUFALLSSEQUENZEN MIT GEPAARTEM ENDE BASIERENDE GENOTYPISIERUNG
GÉNOTYPAGE FONDÉ SUR DES SÉQUENCES ALÉATOIRES À EXTRÉMITÉS APPARIÉES

(30) Priority: 14.01.2011 US 201161432915 P
(43) Date of publication of application: 20.11.2013
(73) Proprietor: Keygene N.V., 6700 AE Wageningen (NL)
(72) Inventor: VAN EIJK, Michael, Josephus, Theresia, NL-6700 AE Wageningen (NL)
(74) Representative: EP&C
(86) International application number: PCT/NL2012/050022
(87) International publication number: WO 2012/096579

(56) References cited:
- WO-A2-2008/041002
- US-A1- 2005 059 022
- US-A1- 2009 269 749
- PAUL D. ETTER ET AL: "Local De Novo Assembly of RAD Paired-End Contigs Using Short Sequencing Reads", PLOS ONE, vol. 6, no. 4, 13 April 2011 (2011-04-13), page E18561, XP55033036, ISSN: 1932-6203, DOI: 10.1371/journal.pone.0018561
- KERSTENS HINDRIK HD ET AL: "Structural variation in the chicken genome identified by paired-end next-generation DNA sequencing of reduced representation libraries", BMC GENOMICS, BIOMED CENTRAL, LONDON, GB, vol. 12, no. 1, 3 February 2011 (2011-02-03), page 94, XP021086511, ISSN: 1471-2164, DOI: 10.1186/1471-2164-12-94
- ZACHARIAH GOMPERT ET AL: "Bayesian analysis of molecular variance in pyrosequences quantifies population genetic structure across the genome of Lycaeides butterflies", MOLECULAR ECOLOGY, vol. 19, no. 12, 1 May 2010 (2010-05-01), pages 2455-2473, XP55033032, ISSN: 0962-1083, DOI: 10.1111/j.1365-294X.2010.04666.x
- KIM YEONG C ET AL: "Evidences showing wide presence of small genomic aberrations in chronic lymphocytic leukemia", BMC RESEARCH NOTES, BIOMED CENTRAL LTD, GB, vol. 3, no. 1, 20 December 2010 (2010-12-20), page 341, XP021091036, ISSN: 1756-0500, DOI: 10.1186/1756-0500-3-341
- PETER J CAMPBELL ET AL: "Identification of somatically acquired rearrangements in cancer using genome-wide massively parallel paired-end sequencing", NATURE GENETICS, NATURE PUBLISHING GROUP, US, vol. 40, no. 6, 1 June 2008 (2008-06-01), pages 722-729, XP002622046, ISSN: 1546-1718, DOI: 10.1038/NG.128 [retrieved on 2008-04-27]
- J. CHEN ET AL: "Scanning the human genome at kilobase resolution", GENOME RESEARCH, vol. 18, no. 5, 1 January 2008 (2008-01-01), pages 751-762, XP55033085, ISSN: 1088-9051, DOI: 10.1101/gr.068304.107
- KORBEL JAN O ET AL: "Paired-end mapping reveals extensive structural variation in the human genome", SCIENCE, AMERICAN ASSOCIATION FOR THE ADVANCEMENT OF SCIENCE, WASHINGTON, DC; US, vol. 318, no. 5849, 1 October 2007 (2007-10-01), pages 420-426, XP002523083, ISSN: 0036-8075, DOI: 10.1126/SCIENCE.1149504
- NATHALIE J. VAN ORSOUW ET AL: "Complexity Reduction of Polymorphic Sequences (CRoPS(TM)): A Novel Approach for Large-Scale Polymorphism Discovery in Complex Genomes", PLOS ONE, vol. 2, no. 11, 1 January 2007 (2007-01-01), pages E1172-E1172, XP55033034, ISSN: 1932-6203, DOI: 10.1371/journal.pone.0001172
- RUAN YIJUN ET AL: "Multiplex parallel pair-end-ditag sequencing approaches in system biology.", WILEY INTERDISCIPLINARY REVIEWS. SYSTEMS BIOLOGY AND MEDICINE 2010 MAR-APR LNKD- PUBMED:20836024, vol. 2, no. 2, March 2010 (2010-03), pages 224-234, XP002680213, ISSN: 1939-005X
- M. J. FULLWOOD ET AL: "Next-generation DNA sequencing of paired-end tags (PET) for transcriptome and genome analyses", GENOME RESEARCH, vol. 19, no. 4, 1 April 2009 (2009-04-01), pages 521-532, XP55015048, ISSN: 1088-9051, DOI: 10.1101/gr.074906.107
- JOHN W. DAVEY ET AL: "Genome-wide genetic marker discovery and genotyping using next-generation sequencing", NATURE REVIEWS GENETICS, vol. 12, no. 7, 17 June 2011 (2011-06-17), pages 499-510, XP55005771, ISSN: 1471-0056, DOI: 10.1038/nrg3012

## Description

### Background to the invention

The majority of marker discovery and genotyping technologies that are currently in use typically rely on two different systems, one for the initial discovery of SNPs and another to subsequently genotype a large number of individuals. This evoked the present applicant to develop a technology for simultaneous sequence-based marker discovery and detection, named random sequence-based genotyping (rSBG). This technology incorporates the high-throughput sequencing capacity of the Illumina GA// and the genome complexity reduction capacities of AFLP^{®} (EP534858). An example thereof is described in WO2007073165 by the present applicant. In contrast to various other genotyping technologies which are often *targeted* (i.e., the SNPs to be detected are selected upfront and targeted by using specific detection probes), rSBG is a *random* approach. In principle all SNPs which are present between the lines and when the specific sequences contained in AFLP templates, will be called (typically after applying stringent mining filters). One of the issues is that when samples are analyzed that are derived from genomes that contain relative large portions of repetitive sequences, such as pepper, identification of polymorphisms between lines becomes increasingly difficult due to the presence of the repetitive sequences.

### Summary of the invention

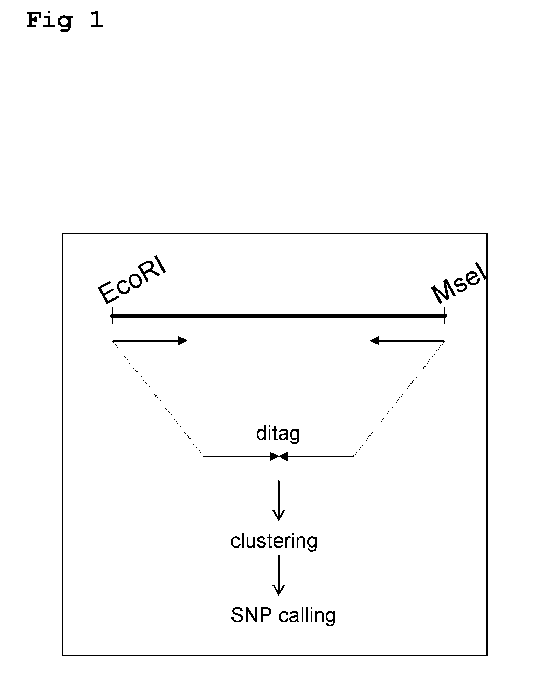

The present inventors have found that improvements can be achieved in the number of polymorphisms scored and genotyped in a plurality of samples, and in particular when samples are used from genomes that are considered as highly repetitive, i.e. contain many repeats, when high throughput sequencing methods are used to sequence both ends of restriction fragments. By employing so called paired-end sequencing approaches, two sets of sequence data (aka sequence reads) are obtained from the same restriction fragment, one from each end of the restriction fragment. By combining these sets, sequence data (sequence reads) from restriction fragments that otherwise would not have been distinguishable from each other, for instance because they originate from a repeats, now become distinguishable. The reason is that the sequence read from the other end of the restriction fragment (often, depending on the restriction enzymes or fragmentation methods used) located hundreds or evened thousand of nucleotide away), is now capable of rendering the combined sequence reads unique (see Fig 1). This now also allows the discovery, detection and genotyping of polymorphisms of samples that are derived from genomes that are highly repetitive. The method of the present invention, in its broadest from, is hence applicable to a broader range of samples than the methods of the prior art as it now also successfully includes highly repetitive samples. The present inventors have found that more SNPs can be discovered and genotyped with this paired-end approach, compared to the separate analysis of the reads of the respective ends of the fragments, i.e. a synergistic effect is obtained that can be attributed to the use of paired-end sequencing in simultaneous discovery and genotyping of SNPs.

### Brief description of the figures

Figure 1: Schematic representation of paired-end random sequence-based genotyping. Ditags are generated form the respective ends of the restriction fragments to achieve maximum separation of repeat sequences for SNP identification.

### Definitions

In the following description and examples, a number of terms are used. In order to provide a clear and consistent understanding of the specification and claims, including the scope to be given such terms, the following definitions are provided. Unless otherwise defined herein, all technical and scientific terms used have the same meaning as commonly understood by one of ordinary skill in the art to which this invention belongs.

Methods of carrying out the conventional techniques used in methods of the invention will be evident to the skilled worker. The practice of conventional techniques in molecular biology, biochemistry, computational chemistry, cell culture, recombinant DNA, bioinformatics, genomics, sequencing and related fields are well-known to those of skill in the art and are discussed, for example, in the following literature references: Sambrook et al.., Molecular Cloning. A Laboratory Manual, 2nd Edition, Cold Spring Harbor Laboratory Press, Cold Spring Harbor, N. Y., 1989; Ausubel et al., Current Protocols in Molecular Biology, John Wiley & Sons, New York, 1987 and periodic updates; and the series Methods in Enzymology, Academic Press, San Diego.

As used herein, the singular forms "a," "an" and "the" include plural referents unless the context clearly dictates otherwise. For example, a method for isolating "a" DNA molecule", includes isolating a plurality of molecules (e.g. 10's, 100's, 1000's, 10's of thousands, 100's of thousands, millions, or more molecules).

Polymorphism: polymorphism refers to the presence of two or more variants of a nucleotide sequence in a population. A polymorphism may comprise one or more base changes, an insertion, a repeat, or a deletion. A polymorphism includes e.g. a simple sequence repeat (SSR) and a single nucleotide polymorphism (SNP), which is a DNA sequence variation, occurring when a single nucleotide: adenine (A), thymine (T), cytosine (C) or guanine (G) - is altered. A variation generally occurs in at least 1% of the population to be considered a SNP. SNPs make up e.g. 90% of all human genetic variations, and occur every 100 to 300 bases along the human genome. Two of every three SNPs substitute Cytosine (C) with Thymine (T). Variations in the DNA sequences of e.g. humans or plants can affect how they handle diseases, bacteria, viruses, chemicals, drugs, etc.

Genotyping refers to the process of determining genetic variations among individuals in a species. The genotype of an organism is the inherited instructions it carries within its genetic code. Not all organisms with the same genotype look or act the same way because appearance and behavior are modified by environmental and developmental conditions. Likewise, not all organisms that look alike necessarily have the same genotype. Single nucleotide polymorphisms (SNPs) are the most common type of genetic variation and by definition are single-base differences at a specific locus that is found in more than 1% of the population. SNPs are found in both coding and non-coding regions of the genome and can lead to different phenotypes, such as the ability to get a disease or to have resistance against it, when found in coding regions. Hence, SNPs are often used as markers for certain diseases or some phenotypes. When found in non-coding regions, SNPs act as markers for evolutionary genomics studies. Related to SNPs are "InDels" or insertions and deletions of nucleotides of varying length. A third type of genetic variation is copy number variation (CNV), which results from having different numbers of copies of a DNA segment in various genomes. In cases where the copy number variation is for an encoded gene, the variation can lead to susceptibility or resistance to disease. Some phenotypes are also dosage-sensitive, and the copy number is responsible for shades of variability among members of a species. For both SNP and CNV genotyping, many methods exist to determine genotype among individuals. The chosen method generally depends on the throughput needs, which is a function of both the number of individuals being genotyped and the number of genotypes being tested for each individual. The chosen method also depends on the amount of sample material available from each individual or sample.

The genotype is the genetic makeup of a cell, an organism, or an individual (i.e. the specific allele makeup of the individual) usually with reference to a specific character or trait under consideration.

A phenotype is the observable characteristics or traits of an organism such as its morphology, development, biochemical or physiological properties, phenology, behavior, and products of behavior. Phenotypes result from the expression of the genes of an as well as the influence of environmental factors and the interactions between the two. Although a phenotype is the ensemble of observable characteristics displayed by an organism, the word phenome is sometimes used to refer to a collection of traits and their simultaneous study is referred to as phenomics.

Phenotyping is determining the phenotype of an organism.

Restriction endonuclease: a restriction endonuclease or restriction enzyme is an enzyme that recognizes a specific nucleotide sequence (target site) in a double-stranded DNA molecule, and will cleave both strands of the DNA molecule at or near every target site, leaving a blunt or a staggered end.

Restriction fragments: the DNA molecules produced by digestion with a restriction endonuclease are referred to as restriction fragments. Any given genome (or nucleic acid, regardless of its origin) will be digested by a particular restriction endonuclease into a discrete set of restriction fragments. The DNA fragments that result from restriction endonuclease cleavage can be further used in a variety of technique.

Tagging: the term tagging refers to the addition of a tag to a nucleic acid sample in order to be able to distinguish it from a second or further nucleic acid sample.

Identifier or identifier tag: a short sequence that can be added to an adaptor or a primer or included in its sequence or otherwise used as label to provide a unique identifier. Such a sequence identifier (tag) can be a unique base sequence of varying but defined length, typically from 4-16 bp. The identifier, or combinations of identifiers can be used for identifying a specific nucleic acid sample or for connecting or associating a DNA product, for instance a fragment or a PCR-product to a sample from which it originates . For instance 4 bp tags allow 4(exp4) = 256 different tags. Using such an identifier, the origin of a sample can be determined upon further processing. In the case of combining processed products originating from different nucleic acid samples, the different nucleic acid samples are generally identified using different identifiers. Identifiers preferably differ from each other by at least two base pairs and preferably do not contain two identical consecutive bases to prevent misreads. The identifier function can sometimes be combined with other functionalities such as adapters or primers.

Tagged restriction fragment: restriction fragment provided with an identifier tag.

Adaptor-ligated restriction fragments: restriction fragments that have been capped by adaptors.

Adaptors: short double-stranded DNA molecules with a limited number of base pairs, e.g. about 10 to about 30 base pairs in length, which are designed such that they can be ligated to the ends of restriction fragments. Adaptors are generally composed of two synthetic oligonucleotides which have nucleotide sequences which are partially complementary to each other. When mixing the two synthetic oligonucleotides in solution under appropriate conditions, they will anneal to each other forming a double-stranded structure. After annealing, one end of the adaptor molecule is designed such that it is compatible with the end of a restriction fragment and can be ligated thereto; the other end of the adaptor can be designed so that it cannot be ligated, but this need not be the case (double ligated adaptors).

Ligation: the enzymatic reaction catalyzed by a ligase enzyme in which two double-stranded DNA molecules are covalently joined together is referred to as ligation. In general, both DNA strands are covalently joined together, but it is also possible to prevent the ligation of one of the two strands through chemical or enzymatic modification of one of the ends of the strands. In that case the covalent joining will occur in only one of the two DNA strands.

Primers: in general, the term primers refer to DNA strands which can prime the synthesis of DNA. DNA polymerase cannot synthesize DNA de novo without primers: it can only extend an existing DNA strand in a reaction in which the complementary strand is used as a template to direct the order of nucleotides to be assembled. We will refer to the synthetic oligonucleotide molecules which are used in a polymerase chain reaction (PCR) as primers.

Synthetic oligonucleotide: single-stranded DNA molecules having preferably from about 10 to about 50 bases, which can be synthesized chemically are referred to as synthetic oligonucleotides. In general, these synthetic DNA molecules are designed to have a unique or desired nucleotide sequence, although it is possible to synthesize families of molecules having related sequences and which have different nucleotide compositions at specific positions within the nucleotide sequence. The term synthetic oligonucleotide will be used to refer to DNA molecules having a designed or desired nucleotide sequence.

Amplification: the term amplification will be typically used to denote the in vitro synthesis of double-stranded DNA molecules, typically using PCR. It is noted that other amplification methods exist and they may be used in the present invention without departing from the gist.

Amplicon: The product of a polynucleotide amplification reaction, namely, a population of polynucleotides that are replicated from one or more starting sequences. Amplicons may be produced by a variety of amplification reactions, including but not limited to polymerase chain reactions (PCRs), linear polymerase reactions, nucleic acid sequence- based amplification, rolling circle amplification and like reactions.

Complexity reduction: the term complexity reduction is used to denote a method wherein the complexity of a nucleic acid sample, such as genomic DNA, is reduced by the generation of a subset of the sample. This subset can be representative for the whole (i.e. complex) sample and is preferably a reproducible subset. Reproducible means in this context that when the same sample is reduced in complexity using the same method, the same, or at least comparable, subset is obtained. The method used for complexity reduction may be any method for complexity reduction known in the art. Examples of methods for complexity reduction include for example AFLP® (Keygene N.V., the Netherlands; see e.g. EP 0 534 858), the methods described by Dong (see e.g. WO 03/012118, WO 00/24939), indexed linking (Unrau, et al., 1994, Gene, 145:163-169), etc. The complexity reduction methods used in the present invention have in common that they are reproducible. Reproducible in the sense that when the same sample is reduced in complexity in the same manner, the same subset of the sample is obtained, as opposed to more random complexity reduction such as microdissection or the use of mRNA (cDNA) which represents a portion of the genome transcribed in a selected tissue and for its reproducibility is depending on the selection of tissue, time of isolation etc.

Selective base, selective nucleotide, randomly selective nucleotide: Located at the 3' end of the primer, the selective base is randomly selected from amongst A, C, T or G (or U as the case may be). By extending a primer with a selective base, the subsequent amplification will yield only a reproducible subset of the adaptor- ligated restriction fragments, i.e. only the fragments that can be amplified using the primer carrying the selective base. Selective nucleotides can be added to the 3'end of the primer in a number varying between 1 and 10. Typically, 1-4 suffice. Both primers (in PCR) may contain a varying number of selective bases. With each added selective base, the subset reduces the amount of amplified adaptor-ligated restriction fragments in the subset by a factor of about 4. this type of complexity reduction is considered random as it does not require or take into account any previous sequence knowledge, it is only based on the selective nucleotide. Typically, the number of selective bases used in the AFLP technology (EP534858) is indicated by +N+M, wherein one primer carries N selective nucleotides and the other primers carries M selective nucleotides. Thus, an Eco/Mse +1/+2 AFLP is shorthand for the digestion of the starting DNA with EcoRI and Msel, ligation of appropriate adaptors and amplification with one primer directed to the EcoRI restricted position carrying one selective base and the other primer directed to the Msel restricted site carrying 2 selective nucleotides. A primer used in AFLP that carries at least one selective nucleotide at its 3' end is also depicted as an AFLP-primer. Primers that do not carry a selective nucleotide at their 3' end and which in fact are complementary to the adaptor and the remains of the restriction site are sometimes indicated as AFLP+0 primers. The term selective nucleotide is also used for nucleotides of the target sequence that are located adjacent to the adaptor section and that have been identified by the use of selective primer as a consequence of which, the nucleotide has become known.

Sequencing: The term sequencing refers to determining the order of nucleotides (base sequences) in a nucleic acid sample, e.g. DNA or RNA. Many techniques are available such as Sanger sequencing and high-throughput sequencing technologies (also known as next-generation sequencing technologies) such as the GS FLX platform offered by Roche Applied Science, and the Genome Analyzer from Illumina, both based on pyrosequencing. Other platforms exist.

High throughput sequencing or next generation sequencing is a sequencing technology that is capable of generating a large amount of reads, typically the order of many thousands (i.e. ten or hundreds of thousands) or millions of sequence reads rather than a few hundred at a time. High throughput sequencing is distinguished over and distinct from conventional Sanger or capillary sequencing. Typically, the sequenced products are the sequenced products themselves which typically have relative short reads, between about 600 and 30 bp. Examples of such methods are given by the pyrosequencing-based methods disclosed in WO 03/004690, WO 03/054142, WO 2004/069849, WO 2004/070005, WO 2004/070007, and WO 2005/003375, by Seo et al. (2004) Proc. Natl. Acad. Sci. USA 101:5488-93. These technologies typical further comprise extensive and elaborate data storage and processing workflows for read assembly etc. The availability of high through[put sequencing requires many conventional workflows and methods for the analysis of genomes to be redesigned to accommodate the type and quality of data that are now produced.

As used herein, 'paired end sequencing' is a method that is based on high throughput sequencing, particular based on the platforms currently sold by Illumina and Roche. Illumina has released a hardware module (the PE Module) which can be installed in the existing sequencer as an upgrade, which allows sequencing of both ends of the template, thereby generating paired end reads. Paired end sequencing can be achieved by reorientation of the strand of the DNA molecule to be sequencing on the carrier in which the sequencing is performed, such as described by Lakdawalla in "Next generation sequencing: towards personalised medicine. Michael Janitz Ed., 2008, Wiley section 2.4. This type of paired end sequencing is typically used for smaller fragments (up to about 1000 bp). Another variant of paired end sequencing is sometimes indicated as mate-pair sequencing wherein, wherein sequencing adapters are ligated to the DNA fragments, the ligated DNAs are digested by type IIs restriction enzymes of which the recognition sequence was included in the adapter, self-circularised, type IIs digested and the resulting paired-ends sequenced. This is particular useful for analysing larger fragments (about > 1000bp) See also Wei et al., "Next generation sequencing: towards personalised medicine. Michael Janitz Ed., 2008, Wiley section 13.2, Fig 13.1 .

A Type-IIs restriction endonuclease is an endonuclease that has a recognition sequence that is distant from the restriction site. In other words, Type IIs restriction endonucleases cleave outside of the recognition sequence to one side. Examples there of are NmeAIII (GCCGAG(21/19) and Fokl, Alwl, Mmel. There are Type IIs enzymes that cut outside the recognition sequence at both sides.

Aligning and alignment: With the term "aligning" and "alignment" is meant the comparison of two or more nucleotide sequence based on the presence of short or long stretches of identical or similar nucleotides. Several methods for alignment of nucleotide sequences are known in the art.

Pooling, as used herein, relates to the combination of a multitude of samples (or artificial chromosomes or clones or subsets of genomes or reproducible complexity reduced genomes) into pools. The pooling may be the simple combination of a number of individual samples into one sample (for example, 100 samples into 10 pools, each containing 10 samples), but also more elaborate pooling strategies may be used. The distribution of the samples over the pools is preferably such that each sample is present in at least two or more of the pools. Preferably, the pools contain from 10 to 10000 samples per pool, preferably from 100 to 1000, more preferably from 250 to 750. It is observed that the number of samples per pool can vary widely, and this variation is related to, for instance, the size of the genome or the number of samples under investigation. Typically, the maximum size of a pool or a subpool is governed by the ability to uniquely identify a sample in a pool, for instance by a set of identifiers. The pools are generated based on pooling strategies well known in the art. The skilled man is capable selecting the optimal pooling strategy based on factors such as genome size, number of samples etc. The resulting pooling strategy will depend on the circumstances, and examples thereof are plate pooling, N-dimensional pooling such as 3D-pooling, 6D-pooling or complex pooling. To facilitate handling of large numbers of pools, the pools may, on their turn, be combined in super-pools (i.e. super-pools are pools of pools of samples) or divided into sub-pools. Other examples of pooling strategies and their deconvolution (i.e. the correct identification of the individual sample in a library by detection of the presence of an known associated indicator (i.e. label or identifier) of the sample in one or more pools or subpools) are for instance described in US 6975943 or in Klein et al. in Genome Research, (2000), 10, 798-807. The pooling strategy is preferably such that every sample in the library is distributed such over the pools that a unique combination of pools is made for every sample. The result thereof is that a certain combination of (sub)pools uniquely identifies a sample.

Clustering: with the term "clustering" is meant the comparison of two or more nucleotide sequences based on the presence of short or long stretches of identical or similar nucleotides and grouping together the sequences with a certain minimal level of sequence homology based on the presence of short (or longer) stretches of identical or similar sequences.

### Detailed description of the invention

In a first aspect, the invention pertains to a method for simultaneous discovery, detection and genotyping of one or more polymorphisms in one or more or a plurality of samples, comprising the steps of:
(a) providing DNA from one or more or a plurality of samples;
(b) reducing the complexity of the sample DNA by digesting the DNA with at least one restriction endonuclease to produce restriction fragments;
(c) providing the restriction fragments of a sample with an identifier tag to produce tagged restriction fragments;
(d) paired-end sequencing of at least part of the tagged restriction fragments;
(e) identify polymorphisms between the samples.

The complexity reduction can be solely based on digestion of the DNA from the sample with one or more restriction enzymes. In certain embodiments, two or more restriction enzymes can be used. To the restriction fragments, adapters can be ligated. The adapters may be ligated to one end or to both ends of the restriction fragments and they may be the same or different. When the restriction fragments are obtained by restriction the DNA with two or more different restriction enzymes, different adapters can be used. Complexity reduction may further be achieved by amplifying the restriction fragments, for instance using primers that are directed against the adapters or part thereof. The primers used in the amplification may further contain parts that are complementary to the remains of the recognition sequence of the restriction enzymes. In certain embodiments, vested technologies such as AFLP® (EP534858) may be used wherein 1-10 randomly selective nucleotides are added at the 3' end of at least one of primers to provide for a reproducible subset of fragments. Other complexity reduction technologies are also possible as long as they are reproducible. Reproducible means in this respect that when the same sample is subjected twice to the complexity reduction, the same subset is obtained and between two samples that substantially the same subset is obtained.

The identifier tag to produce the tagged restriction fragments can be provided in a number of ways. The identifier tag can be provided by:
- ligating tagged adaptors to the restriction fragments to produce tagged adaptor-ligated restriction fragments;
or
amplifying the adaptor-ligated restriction fragments with at least one tagged primer that is complementary to at least part of the adaptor to produce tagged adaptor-ligated restriction fragments.

The adapter may consist solely of the identifier tag or the adapter may contain further functionalities, for instance to allow for selection of (part of) the tagged restriction fragments, for instance to reduce complexity of the sample, for instance on an array.

The identifier tag may also be added in a separate step, before or after adapter ligation, amplification or complexity reduction, as long as per sample a unique tag is provided that links a restriction fragment to the sample form which it originates.

The sequencing step is preferably performed using high throughput sequencing, using paired-end sequencing, including mate-pair sequencing.

In a preferred embodiment of the invention, parts of the sequence of the restriction fragments are determined. Preferably, the sequence of both ends of the restriction fragments are determined and preferably at the same time, i.e. in the same sequencing run. Protocols that provide for such determination of sequences are typically indicated (for GA// and Roche platforms as paired-end sequencing, including mate-pair sequencing as defined herein elsewhere.

Using paired-end sequencing, including mate-pair sequencing typically, sequence information of the two ends of the restriction fragment is obtained. The sequence information from both ends (first read and second read, including the identifier) of the restriction fragment can be combined, leading to a so-called 'ditag'. The ditag contains the combined information of the first and second read which preferably can be linked to the samples using the identifier tag. The identifier tag is preferably associated with (or included in) the first read. The generation of the ditag can be done *in silico.* In a preferred embodiment, one of the reads, preferably the second read, is reverse complemented prior to the generation of the ditag. Reverse complemented means in this respect that the sequence of the read is reversed (for example N1N2N3N4N5N6 becomes N6N5N4N3N2N1).Thus, the ditagin more detail: ID-Read1-Read2(reverse complemented): IDIDIDIDM1M2M3M4M5M6N6N5N4N3N2N1

See also Fig 1 for an illustration of this concept. One part can be obtained from a repetitive sequence, but the other part of the ditag can be derived from another part of the genome sequence, thereby increasing the chance for creating a unique combination of two parts. This allows for the identification of polymorphisms between sequences that otherwise would not have been possible. Current technology allows for 150 nucleotides to be obtained from both sides of the fragment, leading to 300 informative nucleotides. This increases the number of unique combined fragments per sample drastically and hence the number of polymorphisms to be identified. The same technical concept can be performed on other sequencing platforms that allow paired end, including mate-pair sequencing.

High throughput sequencing is preferably based on sequencing by synthesis, pyrosequencing (on a solid carrier) such as platforms provided by Illumina (Ga//, Hiseq, MiSeq) or Roche GS FLX, typically indicated as Next Generation Sequencing. Also technologies indicated as Next Next generation sequencing can be used. Examples thereof are based on sequencing by ligation, hybridisation sequencing, nanopore sequencing (Oxford nanopore technologies or NABsys (US20100096268, US 20100078325, US20090099786)) or as provided by Pacific Biosciences and Ion torrent (Nature 475, Pages: 348-352).

Having obtained the sequence information, the sequences are allocated per sample based on the identifier tag. By clustering (or aligning) the sequences, polymorphisms can be identified between the sequences and hence between the samples. This leads to the identification of SNPs, detection of SNPs and determination of genotypes in multiple samples at the same time. Clustering or alignment can be performed using the conventional technologies in the art.

Methods of alignment of sequences for comparison purposes are well known in the art. Various programs and alignment algorithms are described in: Smith and Waterman (1981) Adv. Appl. Math. 2:482; Needleman and Wunsch (1970) J. Mol. Biol. 48:443; Pearson and Lipman (1988) Proc. Natl. Acad. Sci. USA 85:2444; Higgins and. Sharp (1988) Gene 73:237-244; Higgins and Sharp (1989) CABIOS 5:151-153; Corpet et al. (1988) Nucl. Acids Res. 16:10881-90; Huang et al. (1992) Computer Appl. in the Biosci. 8:155-65; and Pearson et al. (1994) Meth. Mol. Biol. 24:307-31, Atschul et al. (1994) Nature Genet. 6:119-29 present a detailed consideration of sequence alignment methods and homology calculations.

The NCBI Basic Local Alignment Search Tool (BLAST) (Altschul et al., 1990 J Mol Biol. 5;215(3):403-10) is available from several sources, including the National Center for Biological Information (NCBI, Bethesda, Md.) and on the Internet, for use in connection with the sequence analysis programs blastp, blastn, blastx, tblastn and tblastx. It can be accessed at <http://www.ncbi.nlm.nih.gov/BLAST/>. A description of how to determine sequence identity using this program is available at <http: //.ncbi.nlm.nih.gov/BLAST/blast help.html>.

Typically, the alignment is performed on the sequence data that have been trimmed for the adaptors/primer and/or identifiers, i.e. using only the sequence data from the fragments that originate from the nucleic acid sample. Typically, the sequence data obtained are used for identifying the origin of- the fragment (i.e. from which sample), the sequences derived from the adaptor and/or identifier are removed from the data and alignment is performed on this trimmed set.

In an example of the present method, genomic DNA of samples is digested with two restriction enzymes, *E*coRI and *M*seI, and adapters are ligated to the fragments. AFLP complexity reduction can be applied (depending on the complexity of the genome). Finally, the resulting fragments are made suitable for GA// sequencing and sequenced in a paired-end fashion (76 nucleotides each direction). A bioinformatics approach for tag definition and genotype calling is performed and analysis of the resulting data leads to identification of the polymorphism between the samples. The detailed results are described in the examples.

This technology has added value in several ways:
By using the same restriction enzymes as used in making a physical map based on high throughput sequencing of restriction fragments, the sequenced tags and resulting genotypes can be easily linked to the physical map.

By applying paired-end sequencing (i.e. sequencing both ends of the restriction fragments, say the *E*coRI and *M*seI ends of each fragment), followed by aligning only unique combinations of *E*coRI and *M*seI tags, SNP calling and genotyping in duplicated regions is maximized.

Applying robust complexity reduction through AFLP allows pooling large numbers of samples. Thus, in certain embodiments, complexity reduced samples are pooled into pools prior to sequencing.

The technology preferably relies on total genomic DNA.

Throughout this application, various references are cited in parentheses to describe more fully the state of the art to which this invention pertains.

It will be clear that the above description and the figures are included to illustrate some embodiments of the invention, and not to limit the scope of protection. Starting from this disclosure, many more embodiments will be evident to a skilled person which are within the scope of protection and the essence of this invention and which are obvious combinations of prior art techniques and the disclosure of this patent. Hereinafter the invention will be illustrated further by means of non-limiting examples.

### Examples

The goal of the project was to generate a strategy for the analysis of paired-end sequence data in the context of random sequence based genotyping (rSBG). The performance of analyzing the data using a paired-end (ditags) vs. single-end strategy for Arabidopsis was evaluated and compared. For these purposes, reference sequences were generated using a *de novo* assembly strategy with the sequence data from the Illumina GAII NGS platform. Subsequently, the Illumina reads were mapped to the reference sequences. The mapping results were then mined for the presence of SNPs.

The genetic material of the Arabidopsis dataset consisted of two parents, two F1 individuals and 28 offspring from a back cross (BC) population.

The paired-end reads were used to build constructs, named ditags, where they were combined into a single "read". The length of the ditag was the sum of the lengths of each read in the pair. Additionally, before building the ditags the read2 reads were reverse-complemented to enable mapping of the digtag to the reference (genome) sequence. Hence, the final structure of the ditags was: ID tag - read1 - read2 (reverse-complement). The ditags were built before any quality control steps were performed, and the quality control procedures were adapted to filter the ditags with the same criteria used in the filtering of each read file from the paired-end sequence data.

The ID tag was present in both the read1 sand read2 equences from the paired-end sequence data.

EcoRI/Msel Libraries were generated for each of the Arabidopsis samples and sequenced using the Illumina GAII. Quality control approaches were performed for the ditags and for the read1 and read2 files derived from the paired-end sequence data.

The summary statistics for the quality control filtering applied to the Arabidopsis sequence data are indicated in Table 1.

**Table 1 - Descriptive statistics for the Illumina GAII sequence data, in Arabidopsis.**

| | Ditags | Read 1 | Read2 |
|---|---|---|---|
| Initial number of reads | 19,622,319 | 19,622,319 | 19,622,319 |
| Reads without ID tags | 594,273 | 594,273 | 594,273 |
| Reads without *E*coRI restriction enzyme pattern | 3,136,557 | 3,136,557 | n.a. |
| Reads without *M*seI restriction enzyme pattern^{§} | 1,495,914 | n.a. | 4,390,806 |
| Reads containing homopolymer stretches | 18,298 | 39,849 | 17,766 |
| Reads with a significant match against the chloroplast /mitochondria database | 3,438,320 | 3,704,597 | 3,399,068 |
| Reads containing undetermined nucleotides | 25,632 | 32,177 | 23,621 |
| Low quality reads | 32,452 | 54,647 | 138,345 |
| Final number of reads after filtering | 10,880,838 | 12,060,184 | 11,058,405 |
| % of reads with ID tags that passed QC | 55.5 | 61.5 | 56.4 |

| | | | |
|---|---|---|---|
| ^{§}.In the ditags quality control, the presence of the *M*seI pattern was evaluated in the end of the ditag. | | | |

The total number of reads produced in this sequencing lane was 19,622,319. A total of 97% of the reads had the ID tag in the beginning, which indicated that only a small percentage of the sequences were removed because of reads that could not be matched to any sample. After application of all filtering criteria, the number of reads remaining in the dataset ranged from 10.9M (ditags) to 12.1M (read1). The main reasons for removal of reads from the dataset were the absence of the expected restriction enzyme motif (either EcoRI or Msel), and reads that had a significant hit against the chloroplast/mitochondria database.

The comparison of ditags vs. single-end in Arabidopsis was made using CAP3 assemblies (Huang et al. Genome Res. 1999 Sep;9(9):868-77) using the unique reads, for evaluating the performance of analyzing the data as ditags or single-end. The single-end analysis were performed separately for each read file of the paired-end sequence data, and the final results were determined by adding up the numbers obtained in the analysis with each read file. The summary results of this evaluation are indicated in Table 3.

**Table 3 - Summary results for the comparison of the assembly strategy (ditags vs. single-end), in the Arabidopsis sequence dataset (assemblies performed with CAP3 and unique reads).**

| | Ditags | Single-end read 1 | Single-end read2 | Single-end combined |
|---|---|---|---|---|
| Number of reads | 689,512 | 597,878 | 784,782 | 1,382,660 |
| Number of contigs | 31,891 | 38,236 | 43,448 | 81,684 |
| Contigs with SNPs | 4,371 | 1,956 | 1,774 | 3,730 |
| Number of SNPs ^{§} | 8,760 | 3,338 | 2,838 | 6,176 |
| Number of SNPs per contig | 2.0 | 1.7 | 1.6 | 1.7 |
| Number of SNPs ^{¶} (90% genotyping rate) | 2,634 | 1,385 | 997 | 2,382 |
| Number of genotypes (90% genotyping rate) | 78,174 | 41,063 | 29,533 | 70,596 |
| Number of SNPs ^{†} (80% genotyping rate) | 3,346 | 1,791 | 1,352 | 3,143 |
| Number of genotypes (80% genotyping rate) | 96,779 | 51,645 | 38,808 | 90,453 |

| | | | | |
|---|---|---|---|---|
| ^{§} Number of SNPs identified at the default stringency settings ^{¶} At a 90% genotyping rate at least 28 individuals were genotyped ^{†}At a 80% genotyping rate at least 25 individuals were genotyped. | | | | |

The number of SNPs and genotypes, at both genotyping rate thresholds, were higher when the data were analyzed as ditags. This increased performance resulted in the identification of an additional 11% and 7% SNPs and genotypes at the 90% and 80% genotyping rates, respectively. Subsequently, we the number of A, B and H genotypes for each SNP dataset was determined, making use of the backcross population structure available for the Arabidopsis data. Due to the backcross nature of the population, only one homozygote genotype should be observed, hence the number of B genotypes is a good indication of the overall error rate in genotype calling. In addition, the frequency of the A and H genotypes should be approximately 50%, and large deviations from these frequencies are also a sign of problems with genotype calling. The results for the genotype check performed in the Arabidopsis data are included in Table 4

**Table 4 - Results for the genotype check performed in the Arabidopsis data**

| | Ditags | Read 1 | Read2 |
|---|---|---|---|
| Number of SNPs | 2,334 | 1,101 | 1,024 |
| Number of genotypes | 58,452 | 27,647 | 25,078 |
| A genotype | 25,108 | 11,911 | 10,841 |
| % A | 43.0 | 43.1 | 43.2 |
| B genotype | 447 | 252 | 156 |
| % B | 0.8 | 0.9 | 0.6 |
| H genotype | 32,897 | 15,484 | 14,081 |
| % H | 56.3 | 56.0 | 56.1 |

The accuracy of the genotype calling was only performed for the SNPs where the parents were homozygote for alternate alleles. These results confirmed that the genotyping accuracy was high, since the frequency of the B genotype was less than 1% for all strategies tested. Moreover, it also revealed no substantial differences in genotyping accuracy between the three analyses strategies tested, because the frequencies for each genotype class were very similar in all strategies tested.

These results confirmed that the ditag analysis generated a higher number of SNPs and genotypes, without compromising the accuracy of SNP calling and genotyping.

## Claims

1. Method for simultaneous discovery, detection and genotyping of one or more polymorphisms in a plurality of samples, comprising the steps of:
(a) providing DNA from a plurality of samples;
(b) reducing the complexity of the sample DNA by digesting the DNA with at least one restriction endonuclease to produce restriction fragments;
(c) providing the restriction fragments of a sample with at least one identifier tag to produce tagged restriction fragments;
(d) paired-end sequencing at least part of the tagged restriction fragments;
wherein a first sequence read and a second sequence read of a paired end sequence read of a fragment are combined into a ditag, preferably in silico;
(e) identify polymorphisms between the samples.

2. Method according to claim 1 , wherein one of the first or second sequence reads is reverse complemented before combination into a ditag.

3. Method according to claims 1, wherein the identifier tag is provided by:
- ligating tagged adaptors to the restriction fragments to produce tagged adaptor-ligated restriction fragments;
or
- amplifying the adaptor-ligated restriction fragments with at least one tagged primer that is complementary to at least part of the adaptor to produce tagged adaptor-ligated restriction fragments.

4. Method according to claims 1-3, wherein the sequences are allocated to the samples based on the identifier tag.

5. Method according to claims 4, wherein the allocated sequences are compared between samples for the identification of polymorphisms in the sequences between the samples.

6. Method according to claims 1-5, wherein the ditags are compared between the samples.

7. Method according to claims 1-6, wherein the samples are genotyped based on the identified polymorphisms.

8. Method according to claims 1-7, wherein the complexity reduction comprises digestion of the sample DNA with two or more restriction endonucleases to produce restriction fragments.

9. Method according to claims 1-8, wherein adapters are ligated to one or both ends of the restriction fragments to provide adapter ligated fragments.

10. Method according to claim 8 or 9, wherein for each end of a restriction fragment obtained by a different restriction enzyme, a different adapter is ligated.

11. Method according to claim 9 or 10, wherein the complexity reduction further comprises amplifying the adapter-ligated fragments with at least one primer that is at least complementary to part of the adapter.

12. Method according to claim 11, wherein the primer is further complementary to at least part of the remaining part of the recognition sequence of the restriction endonuclease.

13. Method according to claim 12, wherein the primer further contains one or more randomly selective nucleotides at the 3'end of the primer.

14. Method according to claim 12, wherein the primer contains the same one or more randomly selective nucleotides at the 3'end of the primer for the one or more samples.

15. Method according to any of the previous claims wherein sequencing is based on high throughput sequencing, preferably based on pyrosequencing, preferably on a solid carrier or on sequencing by ligation, or nanopore sequencing.

## Patentansprüche

1. Verfahren zum gleichzeitigen Entdecken, Nachweisen und Genotypisieren von einem oder mehreren Polymorphismen in einer Vielzahl von Proben, umfassend die Schritte:
(a) Bereitstellen von DNA aus einer Vielzahl von Proben;
(b) Reduzieren der Komplexität der Proben-DNA durch Verdauen der DNA mit mindestens einer Restriktionsendonuclease, um Restriktionsfragmente herzustellen;
(c) Bereitstellen der Restriktionsfragmente einer Probe mit mindestens einem Identifikator-Tag, um getaggte Restriktionsfragmente herzustellen;
(d) Paired-End-Sequenzieren von mindestens einem Teil der getaggten Restriktionsfragmente;
wobei ein erster Sequenz-Read und ein zweiter Sequenz-Read eines Paired-End-Sequenz-Reads eines Fragments zu einem Ditag kombiniert werden, vorzugsweise in silico;
(e) Identifizieren von Polymorphismen zwischen den Proben.

2. Verfahren gemäß Anspruch 1, wobei einer aus den ersten oder zweiten Sequenz-Reads vor der Kombination zu einem Ditag revers komplementiert wird.

3. Verfahren gemäß Anspruch 1, wobei der Identifikator-Tag bereitgestellt wird durch:
- Ligieren getaggter Adapter an die Restriktionsfragmente, um getaggte, adapterligierte Restriktionsfragmente herzustellen;
oder
- Amplifizieren der adapterligierten Restriktionsfragmente mit mindestens einem getaggten Primer, der zu mindestens einem Teil des Adapters komplementär ist, um getaggte, adapterligierte Restriktionsfragmente herzustellen.

4. Verfahren gemäß den Ansprüchen 1 - 3, wobei die Sequenzen den Proben beruhend auf dem Identifikator-Tag zugeordnet werden.

5. Verfahren gemäß Anspruch 4, wobei zur Identifizierung von Polymorphismen in den Sequenzen zwischen den Proben die zugeordneten Sequenzen zwischen den Proben verglichen werden.

6. Verfahren gemäß den Ansprüchen 1 - 5, wobei die Ditags zwischen den Proben verglichen werden.

7. Verfahren gemäß den Ansprüchen 1 - 6, wobei die Proben beruhend auf den identifizierten Polymorphismen genotypisiert werden.

8. Verfahren gemäß den Ansprüchen 1 - 7, wobei die Komplexitätsreduktion einen Verdau der Proben-DNA mit zwei oder mehr Restriktionsendonukleasen, um Restriktionsfragmente herzustellen, umfasst.

9. Verfahren gemäß den Ansprüchen 1 - 8, wobei Adapter an ein oder beide Enden der Rrestriktionsfragmente ligiert werden, um adapterligierte Fragmente bereitzustellen.

10. Verfahren gemäß Anspruch 8 oder 9, wobei für jedes Ende eines Restriktionsfragments, das durch ein anderes Restriktionsenzym erhalten wird, ein anderer Adapter ligiert wird.

11. Verfahren gemäß Anspruch 9 oder 10, wobei die Komplexitätsreduktion ferner umfasst, die adapterligierten Fragmente mit mindestens einem Primer zu amplifizieren, der mindestens zu einem Teil des Adapters komplementär ist.

12. Verfahren gemäß Anspruch 11, wobei der Primer ferner zu mindestens einem Teil des verbleibenden Teils der Erkennungssequenz der Restriktionsendonuclease komplementär ist.

13. Verfahren gemäß Anspruch 12, wobei der Primer ferner eines oder mehrere zufällig selektive Nucleotide am 3'-Ende des Primers enthält.

14. Verfahren gemäß Anspruch 12, wobei der Primer für die eine oder die mehreren Proben dasselbe eine oder dieselben mehreren zufällig selektiven Nucleotide am 3'-Ende des Primers enthält.

15. Verfahren gemäß einem der vorhergehenden Ansprüche, wobei das Sequenzieren auf High-Throughput-Sequenzieren beruht, vorzugsweise auf Pyrosequenzieren, vorzugsweise auf einem festen Träger, oder auf Sequenzieren durch Ligation oder Nanoporen-Sequenzieren beruht.

## Revendications

1. Procédé pour la découverte, la détection et le génotypage simultanés d'un ou plusieurs polymorphismes dans une pluralité d'échantillons, comprenant les étapes de :
(a) fourniture d'ADN à partir d'une pluralité d'échantillons ;
(b) réduction de la complexité de l'ADN échantillon par digestion de l'ADN avec au moins une endonucléase de restriction pour produire des fragments de restriction ;
(c) fourniture des fragments de restriction d'un échantillon avec au moins un marqueur d'identification pour produire des fragments de restriction marqués ;
(d) séquençage d'extrémités appariées (« paired-end ») d'au moins une partie des fragments de restriction marqués ;
dans lequel une première lecture de séquence et une deuxième lecture de séquence d'une lecture de séquence d'extrémités appariées d'un fragment sont combinées en un dimarqueur (« ditag »), de préférence *in silico :*
(e) identification de polymorphismes entre les échantillons.

2. Procédé selon la revendication 1, dans lequel l'une des première ou deuxième lectures de séquence est changée en inverse complémentaire avant combinaison en un dimarqueur.

3. Procédé selon la revendication 1, dans lequel le marqueur identifiant est fourni par :
- ligature d'adaptateurs marqués aux fragments de restriction pour produire des fragments de restriction ligaturés à des adaptateurs marqués ;
ou
- amplification des fragments de restriction ligaturés à des adaptateurs avec au moins une amorce marquée qui est complémentaire d'au moins une partie de l'adaptateur pour produire des fragments de restriction ligaturés à des adaptateurs marqués.

4. Procédé selon les revendications 1 à 3, dans lequel les séquences sont allouées aux échantillons sur la base du marqueur d'identification.

5. Procédé selon la revendication 4, dans lequel les séquences allouées sont comparées entre échantillons pour l'identification des polymorphismes dans les séquences entre les échantillons.

6. Procédé selon les revendications 1 à 5, dans lequel les dimarqueurs sont comparés entre les échantillons.

7. Procédé selon les revendications 1 à 6, dans lequel les échantillons sont génotypés sur la base des polymorphismes identifiés.

8. Procédé selon les revendications 1 à 7, dans lequel la réduction de la complexité comprend la digestion de l'ADN échantillon avec deux endonucléases de restriction ou plus pour produire des fragments de restriction.

9. Procédé selon les revendications 1 à 8, dans lequel des adaptateurs sont ligaturés à une ou aux deux extrémités des fragments de restriction pour fournir des fragments ligaturés à des adaptateurs.

10. Procédé selon la revendication 8 ou 9, dans lequel pour chaque extrémité d'un fragment de restriction obtenu par une enzyme de restriction différente, un adaptateur différent est ligaturé.

11. Procédé selon la revendication 9 ou 10, dans lequel la réduction de la complexité comprend en outre l'amplification des fragments ligaturés à des adaptateurs avec au moins une amorce qui est au moins complémentaire d'une partie de l'adaptateur.

12. Procédé selon la revendication 11, dans lequel l'amorce est en outre complémentaire d'au moins une partie de la partie restante de la séquence de reconnaissance de l'endonucléase de restriction.

13. Procédé selon la revendication 12, dans lequel l'amorce contient en outre un ou plusieurs nucléotides sélectifs de manière aléatoire à l'extrémité 3' de l'amorce.

14. Procédé selon la revendication 12, dans lequel l'amorce contient le ou les mêmes nucléotides sélectifs de manière aléatoire à l'extrémité 3' de l'amorce pour l'échantillon ou les échantillons.

15. Procédé selon l'une quelconque des revendications précédentes, dans lequel le séquençage est basé sur le séquençage à haut débit, de préférence basé sur le pyroséquençage, de préférence sur un support solide ou sur le séquençage par ligature ou le séquençage à nanopores.
